# EUROPEAN PATENT SPECIFICATION

(11) **EP 1 776 326 B1**
(45) Date of publication and mention of the grant of the patent: **25.01.2012**
(21) Application number: 05712610.4
(22) Date of filing: 03.02.2005
(51) Int. Cl.: C07C 37/86, C07C 39/04

(54) **METHOD FOR REMOVAL OF ACETOL FROM PHENOL**
VERFAHREN ZUR ENTFERNUNG VON ACETOL AUS PHENOL
MÉTHODE DE SÉPARATION DE L"ACÉTOL DU PHÉNOL

(30) Priority: 09.08.2004 US 915723; 16.09.2004 US 944315
(43) Date of publication of application: 25.04.2007
(73) Proprietor: Honeywell International Inc., NJ 07962-2245 (US)
(72) Inventor: WIJESEKERA, Tilak, P., Boothwyn, PA 19061 (US); BHINDE, Manoj, V., Boothwyn, PA 19061 (US)
(74) Representative: Stewart, Lucy Caroline
(86) International application number: PCT/US2005/003228
(87) International publication number: WO 2006/022818

(56) References cited:
- EP-A- 0 656 342
- EP-A- 1 024 128
- WO-A-2004/072009
- GB-A- 883 746
- GB-A- 933 723
- US-A- 4 334 107
- US-B1- 6 486 365

## Description

### FIELD OF THE INVENTION

The present invention relates to the field of the production of high purity phenol. More particularly, the present invention relates to the removal of acetol from phenol to obtain desired high purity.

### BACKGROUND OF THE INVENTION

The process commonly practiced for the production of phenol involves the oxidation of cumene to cumene hydroperoxide; followed by its acid catalyzed decomposition to phenol and acetone. Isolation of phenol from the reaction product involves the neutralization of the acid catalyst, followed by a series of distillation and separation steps. The lower boiling components such as acetone, unreacted cumene as well as α-methylstyrene (AMS) are first recovered from the crude product by distillation. The remaining material is introduced into a phenol recovery column in which phenol is distilled away from the higher boiling impurities. Depending on the distillation procedures used to recover acetone, cumene and AMS, the distilled phenol may contain minor quantities of impurities such as mesityl oxide (MO), acetol (hydroxyacetone) and other aliphatic carbonyl compounds, olefinic compounds, acetophenone, cumylphenols and 2- and 3-methylbenzofuran (MBF) in addition to residual amounts of acetone, cumene and AMS. Such impurities are undesirable in phenol used in certain applications such as in the manufacture ofbisphenol-A.

MBF is a particularly undesirable contaminant of phenol that is used for certain applications such as in the production of bisphenol-A, a precursor to polycarbonate resins. Due to similar volatility, MBF cannot be separated from phenol by fractional distillation. U.S. Patents 5,064,507 and 4,857,151 describe a process of distillation in the presence of water (also called steam stripping) to reduce MBF in phenol. However, due to the high energy costs and the necessity to use large distillation columns, this process is expensive in terms of capital investment and operating costs. U.S. Patent 5,414,154 describes the use of a strong acid ion exchange resin to reduce the level of MBF by converting it to higher boiling compounds. U.S. Patent 5,414,154 also showed that the effectiveness of MBF removal by resin treatment increases with an increase in temperature.

Although strong acid ion exchange resins also remove carbonyl compounds from phenol on contact, acetol reacts with phenol to produce more MBF. U.S. Patent 5,414,154 teaches the necessity to remove acetol from phenol (e.g. by treatment with an amine) prior to contact with the resin to remove MBF.

Although effective, amine treatment involves the use of an expensive reagent, which must subsequently be purged from the phenol stream.

Both U.S. Patents 3,810,946 and 6,489,519 disclose treatment of a phenol stream containing acetol with an acid or acid resin to remove acetol. British Patent 0 865 677 discloses a process for removing acetol from a phenol stream wherein the phenol stream is heated in the presence or absence of a catalyst. However, in all of these patents, acetol is removed by reacting it with phenol to form MBF, which is subsequently purged from the phenol stream.

European Patent 0 004 168 and U.S. Patent 4,857,151 disclose distillation processes to remove acetol from phenol streams. However, these methods involve the use of capital intensive distillation apparatus.

EP 1 024 128 A describes a process for removing hydroxyacetone from phenol containing the same, which process comprises treating the phenol with bases in the vapor-phase.

U.S. Patent 6,486,365 B1 discloses a process for the removal of acetol from phenol streams by heating in the presence of a hydrotalcite-type catalyst to convert acetol into higher boiling compounds followed by distillation. Similarly, EP 0656 342 A discloses a process for the removal of acetol from phenol streams by heating in the presence of a zeolite catalyst for 5 hours and subsequent rectification.

There remains a need for an efficient, low cost method for the removal of acetol from a phenol stream that does not adversely affect phenol yields by formation of significant amount of additional MBF.

### SUMMARY OF THE INVENTION

The present invention provides efficient, low cost methodologies for the removal of acetol from a phenol stream.

In the present invention, a method for the efficient, low cost removal of acetol from phenol comprises of heating the phenol stream at a temperature greater than about 175°C to convert acetol into higher boiling compounds other than methylbenzofuran. This is be carried out with added alkali metal hydroxide. The phenol stream is then distilled to separate phenol from the higher boiling compounds.

In the present invention, acetol is effectively removed without reacting a large portion of the acetol with phenol, thus the present method results in reduced MBF formation and improved phenol yields.

### BRIEF DESCRIPTION OF THE DRAWINGS

- Figure 1-: Illustrates the removal of acetol from a phenol stream by heating in a closed system at 198°C.
- Figure 2-: Illustrates the removal of acetol from a phenol stream by heating in a closed system at 198°C in the presence of 266ppm of 50% caustic.

### DESCRIPTION OF THE INVENTION

It has been discovered that acetol can be removed efficiently from phenol at a low cost while minimizing the formation of methylbenzofuran (MBF). The treatment to remove acetol involves a treatment of distilled phenol containing acetol at elevated temperature with a small amount of caustic. By this treatment, acetol present in the crude phenol is converted primarily to high boiling products other than MBF. These high boiling products can then be separated from phenol via distillation.

The removal of acetol is key to the subsequent efficient removal of MBF from the crude phenol. As is known, at the high temperatures and acidic conditions used to convert MBF to products separable from phenol, acetol reacts with phenol to produce more MBF. This reaction has the dual effect of reducing phenol recovery and making the removal of MBF from the product less efficient.

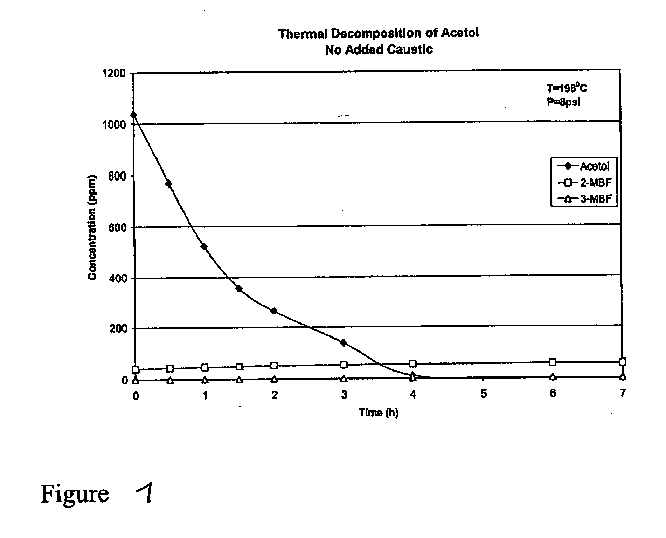

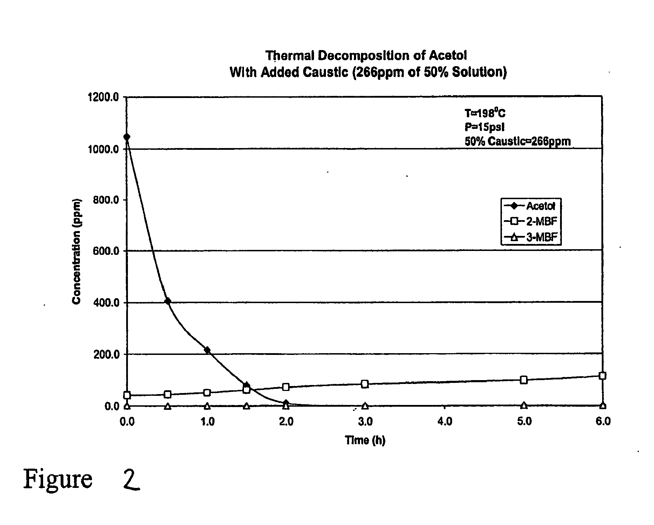

In the invention, a distilled phenol stream containing acetol is heated in a closed system at elevated temperature to convert acetol to higher boiling compounds other than MBF. The heat treatment is carried out with the addition of a small amount of caustic. If the heat treatment is carried out without the addition of caustic, the pH of the phenol stream should be above 2, preferably above 2.5. In general, this is the pH of the distilled phenol stream from the neutralized reaction product. Lower pH may lead to additional amounts of MBF. The caustic is preferably added as a concentrated solution, e.g. 50 percent by weight. Again, the phenol is separated from the higher boiling compounds via distillation. The temperatures used for the heat treatment will be at least 175 to 225° C. The treatment time, temperature and the amount of caustic used (if required) will vary based on thy quantity of acetol to be removed from the phenol stream. As illustrated in **Figure 1**, in an exemplary treatment 1036ppm of acetol was reduced to less than 10 ppm by heating at 198°C for about 4 hours without the addition of any caustic. In this example only 1 percent of the acetol was converted into MBF. As illustrated in **Figure 2**, in the presence of 266ppm of 50% caustic, 1045ppm of acetol was reduced to less than 10ppm in only about 2 hours. However, a slightly higher amount of acetol (~1.6%) was converted into MBF. It should be pointed out that the efficient removal of acetol at elevated temperatures requires a low water content in the phenol stream being treated, for example less than 1.5 percent based on phenol. Preferably, the water content is reduced to about 0.1 percent by weight. Various methods for reducing the water content of organic streams, and phenol in particular, are known in the art. Other examples reducing acetol from 1800 ppm are shown in Table 1 below.

**TABLE 1**

| **Acetol Removal By Heat Treatment With Added Caustic** | | | |
|---|---|---|---|
| ***1800 ppm Acetol to 10 ppm*** | **50% Caustic Concentration** | | |
| **Temperature** | | | |
| | **260 ppm** | **525 ppm** | **1100 ppm** |
| 175° C | >7 hr | | |
| 190° C | 5 hr | 5 hr | |
| 198° C | 4.5 hr | 3.5 hr | 2.3 hr* |

| | | | |
|---|---|---|---|
| *2.75 hr at 1.5% water concentration | | | |

Following the treatment to remove acetol, the phenol is distilled from the higher boiling compounds, and can be passed to an acidic resin treatment at elevated temperature to remove MBF, as disclosed in U.S. Patents 5,414,154 and 6,388,144 B1.

The present method to remove acetol from phenol has the advantage of being more cost efficient than prior art methods that involved the use of distillation apparatus, such as super splitter columns and prior art methods that used expensive amines. In addition, the present method has the advantage of producing less MBF from the acetol being removed, than other prior art methods, which utilized high concentrations of caustic or relied on multiple high temperature treatments with acidic resins.

## Claims

1. A process for removing acetol from a phenol stream, said method comprising:
providing a phenol stream containing acetol and having a water content of 1.5 weight percent or less based on phenol,
adding an alkali metal hydroxide to the phenol stream to obtain an alkali metal concentration of 600 ppm by weight or less based on phenol,
heating said phenol stream at a temperature of greater than 175°C to convert acetol into higher boiling compounds other than methylbenzofuran, and
distilling said phenol stream to separate phenol from said higher boiling compounds.

2. The process according to claim 1 wherein said alkali metal hydroxide is sodium hydroxide.

3. The process of claim 1, wherein the phenol stream is heated to a temperature of 175°C to 225°C.

4. The process of claim 1, wherein said phenol stream contains up to 1800 ppm acetol.

5. The process of claim 1, wherein said distillation is a flash distillation.

6. The process to claim 1, wherein said heating is carried out for a period of 2 to 8 hours.

7. The process according to claim 1, wherein said phenol stream is treated to reduce the content of water to 0.1 percent by weight or less based on phenol prior to said adding of an alkali metal hydroxide.

## Patentansprüche

1. Verfahren zur Entfernung von Acetol aus einem Phenolstrom, bei dem man:
einen Phenolstrom, der Acetol enthält und einen Wassergehalt von 1,5 Gewichtsprozent oder weniger, bezogen auf Phenol, aufweist, bereitstellt,
durch Zugabe eines Alkalimetallhydroxids zu dem Phenolstrom eine Alkalimetallkonzentration von 600 Gew.-ppm oder weniger, bezogen auf Phenol, einstellt,
durch Erhitzen des Phenolstroms auf eine Temperatur von mehr als 175°C Acetol in höhersiedende Verbindungen, die von Methylbenzofuran verschieden sind, umwandelt und
durch Destillation des Phenolstroms Phenol von den höhersiedenden Verbindungen trennt.

2. Verfahren nach Anspruch 1, bei dem es sich bei dem Alkalimetallhydroxid um Natriumhydroxid handelt.

3. Verfahren nach Anspruch 1, bei dem man den Phenolstrom auf eine Temperatur von 175°C bis 225°C erhitzt.

4. Verfahren nach Anspruch 1, bei dem der Phenolstrom bis zu 1800 ppm Acetol enthält.

5. Verfahren nach Anspruch 1, bei dem es sich bei der Destillation um eine Flash-Destillation handelt.

6. Verfahren nach Anspruch 1, bei dem man das Erhitzen über einen Zeitraum von 2 bis 8 Stunden durchführt.

7. Verfahren nach Anspruch 1, bei dem man den Phenolstrom vor der Zugabe des Alkalimetallhydroxids zur Verringerung des Wassergehalts auf 0,1 Gewichtsprozent oder weniger, bezogen auf Phenol, behandelt.

## Revendications

1. Procédé permettant d'éliminer de l'acétol dans un courant de phénol, ledit procédé comprenant les étapes consistant à :
- se procurer un courant de phénol contenant de l'acétol et ayant une teneur en eau d'au plus 1,5 pour cent en poids par rapport au phénol,
- ajouter un hydroxyde de métal alcalin au courant de phénol pour obtenir une concentration en métal alcalin d'au plus 600 ppm en poids par rapport au phénol,
- chauffer ledit courant de phénol à une température de plus de 175°C pour convertir l'acétol en composés de point d'ébullition plus élevé autres que le méthyl-benzofurane, et
- distiller ledit courant de phénol pour séparer le phénol desdits composés de point d'ébullition plus élevé.

2. Procédé selon la revendication 1, dans lequel ledit hydroxyde de métal alcalin est l'hydroxyde de sodium.

3. Procédé selon la revendication 1, dans lequel le courant de phénol est chauffé à une température de 175°C à 225°C.

4. Procédé selon la revendication 1, dans lequel ledit courant de phénol contient jusqu'à 1800 ppm d'acétol.

5. Procédé selon la revendication 1, dans lequel ladite distillation est une distillation éclair.

6. Procédé selon la revendication 1, dans lequel ledit chauffage est réalisé pendant une durée de 2 à 8 heures.

7. Procédé selon la revendication 1, dans lequel ledit courant de phénol est traité de manière à réduire la teneur en eau à au plus 0,1 pour cent en poids par rapport au phénol avant ledit ajout d'un hydroxyde de métal alcalin.
